# EUROPEAN PATENT APPLICATION

(11) **EP 2 540 691 A1**
(43) Date of publication of application: **02.01.2013**
(21) Application number: 11171919.1
(22) Date of filing: 29.06.2011
(51) Int. Cl.: C07C 2/76, C07C 15/02, C07C 15/27, C07D 213/06

(54) **Method and catalyst for the alkylation of aromatic compounds with light alkanes**

(71) Applicant: Stamicarbon B.V. acting under the name of MT Innovation Center, 6135 KW Sittard (NL); Universität Stuttgart, 70174 Stuttgart (DE)
(72) Inventor: The designation of the inventor has not yet been filed
(74) Representative: Jansen, Cornelis Marinus

(57) **Abstract**

Disclosed is a process for the direct alkylation of aromatic compounds with alkanes, particularly with light alkanes of 1-12 carbon atoms. To this end a judicious catalyst combination is provided. The composition comprises palladium as a catalytically active metal, or another metal of the platinum group. The composition comprises zinc as a promoter, or a metal such as tin having a comparable promoting action. The metals are contained in a zeolite support, or a similar support of a metal organic framework type or a silico alumino phosphate type.

## Description

### Field of the invention

The invention pertains to a method for the alkylation of aromatic compounds with alkanes. Particularly, the invention relates to the direct alkylation of aromatic hydrocarbons with short-chain alkanes, having a chain length of from 1 to 12 carbon atoms.

### Background of the invention

Alkylated aromatics, e.g. ethylbenzene and ethyltoluene, find widespread usage. In conventional processes to produce such alkyl aromatics, an aromatic hydrocarbon is alkylated with a reactive agent such as olefin, alkyl halide or alkyl alcohol. Processes for the direct alkylation of aromatics with alkanes are virtually non-existent. Yet, this would be desired since regular alkylation agents, such as alkenes, are expensive. It would be desired for the alkylation of aromatics to be possible with alkanes instead of alkenes because alkanes directly occur in nature in the form of natural gas, whereas alkenes have to be made from alkanes. Thus, alkanes are cheaper than alkenes, and a process step can be saved. However, to be able to use alkanes as alkylating agents, a very active and selective catalyst is needed since the reaction is severely limited by thermodynamics.

An existing process is the "M-Forming" process. This starts with longer alkanes, cracks them and uses then the olefinic fragments again as alkylating agents for aromatics alkylation. Similarly, US 4,899,008 refers to a direct catalytic alkylation of mononuclear aromatics with lower alkanes. Therein an acid H-ZSM-5 catalyst is used. The main alkylation products are not direct alkylation products but products formed from cracked propane. Since cracking of propane produces methane and ethene, it is likely that ethene acted as alkylating agent.

A reference on the direct alkylation of aromatics with alkanes is WO 99/59942. The reaction is catalyzed by a molecular sieve catalyst comprising incorporated metal. Herein a hydrocarbon feed containing an aromatic hydrocarbon is contacted with an alkane of at least 15 carbon atoms. Reactions conditions for the conversion of such longer alkanes, however, are not normally suitable for light alkanes. The problem with longer alkanes is their high reactivity, particularly towards cracking. The problem with light alkanes, such as those having chain lengths of from 1 to 12 carbon atoms, and more particularly from 1 to 8 carbon atoms, is that they are difficult to activate.

Hence, a demand exists in the art to provide a process for the direct alkylation, with light alkanes, of aromatic compounds.

### Summary of the invention

In order to better address one or more of the foregoing desires, the invention presents, in one aspect, a process for the alkylation of an aromatic compound, comprising contacting the aromatic compound with an alkane of from 1 to 12 carbon atoms, under elevated temperature, in the presence of a catalyst composition comprising a catalytically active metal and a promoter metal on a support selected from the group consisting of synthetic zeolites, metal organic frameworks, silico alumino phosphate molecular sieves, and mixtures thereof, wherein the catalytically active metal is selected from the group consisting of palladium, platinum, ruthenium rhodium, osmium, iridium, and combinations thereof, and the promoter is selected from the group consisting of zinc, tin, gallium, germanium, indium, lead, bismuth, rhenium, and combinations thereof.

In another aspect, the invention provides the use of a catalyst composition as defined above, for the activation of an alkane of from 1 to 12 carbon atoms towards the direct alkylation of an aromatic compound.

### Brief description of the drawings

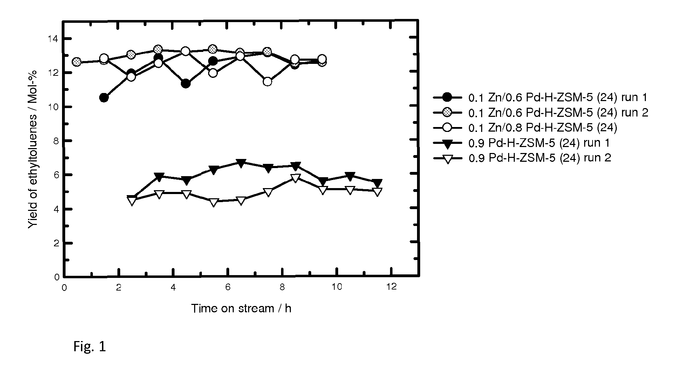

Fig. 1 is a graph representing the yield of ethyltoluenes over time, upon direct alkylation of toluene with ethane. Depicted is the result of a process under the influence of three catalyst compositions of the invention. The measurement points hereof are represented by black, white and gray bullets. The graph includes a comparison with a catalyst composition not according to the invention. The measurement points hereof are indicated with black and white triangles.

### Detailed description of the invention

In a broad sense, the invention is based on the judicious insight that a metal catalyst that has a strong hydrogenating / dehydrogenating activity, in combination with a promoter metal, is able to achieve the activation of light alkanes towards the direct alkylation of aromatic compounds. The combination of the catalyst and the promoter is presented on a porous support, which is a synthetic zeolite or a recognized alternative having a similar molecular sieve characteristic, such as a metal organic framework (MOF) or a silico alumino phosphate molecular sieve (SAPO).

The zeolite-type support is desired for the presence of acidic sites. Amongst known zeolites, ZSM-5 and the like are suitable to prevent coking and to suppress thermodynamically favored reactions. Thus, preferred zeolites include ZSM-5, ZSM-11, ZSM-12, ZSM-23, ZSM-35, and combinations thereof. As is known to the skilled person, in current times alternatives exist that can be formed into molecular sieves having characteristics similar to those of zeolites. These alternatives include so-called metal organic frameworks (MOF's) and silico alumino phosphates.

Preferred supports for use in the present invention are selected from the group of synthetic zeolites and similar materials, such as SAPOs, MOFs or the like, having the characteristics of, e.g., ZSM-5, ZSM-11, ZSM-12, ZSM-23, ZSM-35, and having a spaciousness index less than or equal to 20 and a modified constraint index of 1 to 14. The spaciousness index and the modified constraint index are known methods to characterize zeolites and zeolite-type materials. These terms are well-defined in the art. Reference can be made, *inter alia,* to the "Handbook of Porous Solids", F. Schüth, K.S.W. Sing, J. Weitkamp (eds.), Wiley-VCH, 2002. Particularly for zeolites, see, e.g., pages 699, for SAPOs, e.g., pp. 815, for MOFs, e.g., pp. 1190, and for spaciousness index and modified constraint index e.g., pp. 1015.

The support material desirably has acidic sites. On this basis, good results can be obtained with medium Si/A1 molar ratios. However, for the optimal working of the promoter, it is believed that reasonable ion exchange capacities are desired, which would imply reasonably low Si/Al molar ratios. All in all, it is preferred for the zeolites to have SilAl molar ratios between 2 and 100, preferably between 5 and 50, more preferably between 10 and 35, and most preferably between 15 and 30.

The preferred combination of metal catalyst and promoter is palladium, as the metal, and zinc, as the promoter. The molar ratio of promoter metal to mainly active metal generally is between 0.01 and 5, preferably between 0.1 and 1.5, most preferably between 0.1 and 0.5.

Without wishing to be bound by theory, the inventors believe that, besides palladium, other metal catalyst are suitable if they possess a strong hydrogenation / dehydrogenation activity. Preferred catalyst metals in this respect are the platinum group metals. These include platinum, palladium, ruthenium, rhodium, osmium, and iridium. The most preferred catalytically active metal is palladium.

With reference to the favourable promoter activity of zinc, other metals with similar promoter activities include gallium, tin, germanium, indium, lead, bismuth, and rhenium.

Combinations of catalytically active metals as well as combinations of promoter metals can also be used.

The catalyst composition of the invention generally comprise 0.1 wt.% to 5 wt.% of the active metal, preferably 0.2 wt.% to 1 wt. %, most preferably between 0.4 wt.% and 0.9 wt.%. With the addition of the promoter, the content of the mainly active metal can be reduced.

The catalyst composition of the invention serves to activate light alkanes towards the direct alkylation of aromatic compounds. Light alkanes, as used in the present invention, are aliphatic hydrocarbons having chain engths of 1 to 12 carbon atoms, preferably and more particularly from 1 to 8 carbon atoms, more preferably from 1 to 6 carbon atoms. These alkanes can be linear or branched, with n-alkanes being preferred. Still more preferred alkanes have chain lengths of 2 to 4 carbon atoms. Ethane and propane are the most preferred. With light alkanes, and particularly with ethane and propane, a particular challenge has been overcome by presenting a catalyst composition that is actually suitable to support a direct alkylation reaction of aromatic compounds.

The source of the alkanes used in the alkylation reaction is not of particular relevance. E.g., the process of the invention can also be carried out using light alkanes that are formed from prior cracking of higher alkanes. However, it will be understood that in order to fully enjoy the benefits of the invention, it is preferred to employ light alkanes provided from direct, existing sources of such alkanes.

The most preferred catalyst composition comprises palladium as the catalytically active metal and zinc as the promoter metal. In a most preferred embodiment of the palladium/zinc catalyst composition, wherein zinc is believed to act optimally as a promoter, the Si/A1 molar ratio of the zeolite is between 10 and 35.

Aromatic compounds to be alkylated by the processes of the present invention preferably comprise one to three phenyl rings. Other rings, such as five-membered or seven-membered rings fused into an aromatic ring system are conceivably also alkylated by the process of the invention. The aromatic compounds can comprise full carbon rings, but also heterocyclic aromatic compounds are included. Preferred aromatic compounds are selected from the group consisting of benzene, toluene, other alkyl aromatics, phenol, anthracene, phenanthrene, and pyridine..

In the process of the invention, as in largely any catalytic alkylation process, temperature, and preferably also pressure, will be elevated as compared to room temperature. Preferably, the reaction is conducted at a temperature of 200°C to 500 °C, more preferably 320°C to 400°C.

The pressure employed will generally depend on the type of reactor used. Preferred pressures are within a range of from 1 bar to 200 bar, more preferably 5 bar to 50 bar, and most preferably 7 bar to 20 bar.

In the preferred embodiment of a combination of palladium and zinc, it is believed that zinc serves to dilute the palladium, and thus modifies the activity and selectivity of the catalyst into the direction desired for the direct alkylation of aromatic compounds.

Whilst similar catalysts may already have been used for other applications, e.g., the dehydrogenation of alkanes, this is not the case for the alkylation of aromatics with alkanes, particularly with light alkanes. The use of zinc allows considerably higher yields of the desired alkyl aromatics, i.e., about 12% instead of 5% during the alkylation of toluene with ethane in a fixed-bed reactor at 24 bar and 350°C (see Fig. 1).

The invention will further be described with respect to non-limiting examples and with reference to a figure. The invention is not limited thereto but only by the claims. Where the term "comprising" is used in the present description and claims, it does not exclude other elements or steps. Where an indefinite or definite article is used when referring to a singular noun, e.g., "a" or "an", "the", this includes a plural of that noun unless something else is specifically stated.

### Example 1

### Preparation of the catalyst

Palladium ion exchange was carried out by adding drop wise under stirring an aqueous solution of 0.304 g Pd(NH₃)₄Cl₂ (40.62 wt.-% Pd, ChemPur) in 250 ml demineralized water to a suspension of 9.446 g (dry mass) zeolite (Si/A1 molar ratio of the zeolite is between 10 and 35) in 250 ml demineralized water. The mixture was stirred at room temperature for 24 hours, filtered and dried at 353 K for another 24 h.

It will be understood that the amounts of Pd salt, water and zeolite can be varied. It is also possible to save Pd salt by not filtering the solution but carefully evaporating the water.

The catalyst was then calcined at 823 K in nitrogen for another 24 h and cooled to room temperature. 2.613 g (dry mass) zeolite were suspended in 25 ml demineralized water and 0.013 g of zinc acetate (C₄H₆O₄ Zn ·2H₂O, Fluka 99.0%) were added. Then the water was carefully removed in a rotary evaporator, thereby impregnating the catalyst with the zinc salt. Afterwards, the catalyst was dried again at 353 K for 24 h.

### Example 2

### Catalytic Experiments

For the catalytic experiments, the zeolite powder was pressed without a binder, crushed and sieved to get a particle size between 0.2 and 0.3 mm. The catalyst was activated in situ, prior to starting the experiment. To achieve a high dispersion of the noble metal, 0.5 g of the catalyst were first heated in flowing synthetic air (150 cm³ min⁻¹) at a rate of 0.25 K min-¹ to a final temperature of 573 K, then it was switched to nitrogen (150 cm³ min⁻¹) and heated with a rate of 1.7 K min⁻¹ to a final temperature of 623 K. Afterwards the catalyst was reduced under a constant stream of hydrogen (150 cm³ min⁻¹) at 623 K for 4 h.

Catalytic experiments were performed in a flow-type apparatus with a fixed-bed reactor from stainless steel. Ethane (99.95 vol.-%, Westfalen AG) and nitrogen (99.999 vol.-%, Westfalen AG) were fed with an *ṅ* nitrogen / *ṅ* ethane ratio of approximately 4 through a toluene (> 99.9 %, Merck) saturator containing Chromosorb P-NAW (Macherey-Nagel). Nitrogen was used as an internal standard but also to ensure that a relatively low *ṅ* ethane / *ṅ* toluene feed ratio of 5±1 could be achieved at the high pressure applied. The reaction was carried out at a total pressure of 24 bar and a reaction temperature of (350±2) °C. The WHSV (toluene and ethane) was 1.0 h-¹. Product analysis was achieved using an on-line sampling system, a capillary gas chromatograph and a CP-PoraPLOT Q column (length: 30 m, inner diameter: 0.32 mm, film thickness: 20 µm, Chrompack). Two detectors in series were employed, namely, a thermal conductivity detector followed by a flame ionization detector. Correction factors for the two detectors were determined separately. With ethane as tie substance, the results from both detectors were combined. From the mass and molar flows, the selectivities of all products were calculated in mol%. The yields were determined from the selectivities and the toluene conversion.

In Fig.1 a graphic representation is given of the yield of ethyltoluenes during the alkylation of toluene with ethane on zeolite catalysts in a fixed-bed reactor (pressure: 24 bar; reaction temperature: 350 °C).

## Claims

1. A process for the alkylation of an aromatic compound, comprising contacting the aromatic compound with an alkane of from 1 to 12 carbon atoms, under elevated temperature, in the presence of a catalyst composition comprising a catalytically active metal and a promoter metal on a support selected from the group consisting of synthetic zeolites, metal organic frameworks, silico alumino phosphate molecular sieves, and mixtures thereof, wherein the catalytically active metal is selected from the group consisting of palladium, platinum, ruthenium rhodium, osmium, iridium, and combinations thereof, and the promoter is selected from the group consisting of zinc, tin, gallium, germanium, indium, lead, bismuth, rhenium, and combinations thereof.

2. A process according to claim 1, wherein the catalytically active metal is palladium and the promoter metal is zinc.

3. A process according to claim 1 or 2, wherein the support is selected from the group of synthetic zeolites and similar materials, such as SAPO_{S}, MOFs or the like, having the characteristics of ZSM-5, ZSM-11, ZSM-12, ZSM-23, ZSM-35, and having a spaciousness index less than or equal to 20 and a modified constraint index of 1 to 14.

4. A process according to claim 3, wherein the support is selected from the group consisting of ZSM-5, ZSM-11, ZSM-12, ZSM-23, ZSM-35, and combinations thereof.

5. A process according to any one of the preceding claims, wherein the support is a zeolite having an Si/Al molar ratio between 2 and 100.

6. A process according to claim 5, wherein the support is a zeolite having an Si/Al molar ratio between 5 and 50, preferably between 15 and 30.

7. A process according to any one of the preceding claims, wherein the molar ratio of the promoter metal to the catalytically active metal is between 0.01 and 5.

8. A process according to claim 7, wherein the molar ratio is between 0.1 and 1.5, preferably between 0.1 and 0.5.

9. A process according to any one of the preceding claims, comprising 0.1 wt.% to 5 wt.% of the catalytically active metal.

10. A process according to claim 9, comprising 0.2 wt.% to 1 wt.% of the catalytically active metal, preferably between 0.4 wt.% and 0.9 wt.%.

11. A process according to any one of the preceding claims, wherein the alkane has 2 to 4 carbon atoms.

12. A process according to any one of the preceding claims, wherein the aromatic compound is selected from the group consisting of benzene, toluene, phenol, anthracene, phenanthrene, and pyridine.

13. A process according to any one of the preceding claims, wherein the reaction is conducted at a temperature of 200°C to 500 °C, preferably 320°C to 400°C.

14. A process according to any one of the preceding claims, wherein the reaction is conducted under a pressures within a range of from 1 bar to 200 bar.

15. A process according to claim 14, wherein the pressure is 5 bar to 50 bar, preferably 7 bar to 20 bar.

16. The use of a catalyst composition as defined in any one of the claims 1 to 10, for the activation of an alkane of from 1 to 12 carbon atoms towards the direct alkylation of an aromatic compound.
